(19) Europäisches Patentamt
European Patent Office
Office européen des brevets

(11) Veröffentlichungsnummer : **0 387 531 B1**

(12) # EUROPÄISCHE PATENTSCHRIFT

(45) Veröffentlichungstag der Patentschrift : **19.05.93 Patentblatt 93/20**

(51) Int. Cl.$^5$ : **C07C 317/32,** C07C 315/04

(21) Anmeldenummer : **90102766.4**

(22) Anmeldetag : **13.02.90**

(54) **Verfahren zur Herstellung von beta-Chloräthylsulfonyl-arylisocyanaten.**

(30) Priorität : **16.02.89 DE 3904591**

(43) Veröffentlichungstag der Anmeldung : **19.09.90 Patentblatt 90/38**

(45) Bekanntmachung des Hinweises auf die Patenterteilung : **19.05.93 Patentblatt 93/20**

(84) Benannte Vertragsstaaten : **BE CH DE FR GB IT LI**

(56) Entgegenhaltungen :
**EP-A- 0 265 857**
**DE-A- 2 054 198**
**DE-B- 1 289 930**

(73) Patentinhaber : **HOECHST AKTIENGESELLSCHAFT**
**Postfach 80 03 20**
**W-6230 Frankfurt am Main 80 (DE)**

(72) Erfinder : **Steuernagel, Hans Helmut, Dr.**
**An den Römergärten 1**
**W-6233 Kelkheim (Taunus) (DE)**

EP 0 387 531 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein vorteilhaftes Verfahren zur Herstellung von β-Chloräthylsulfonyl-arylisocyanaten der allgemeinen Formel (1)

$$Cl\text{-}H_2C\text{-}H_2C\text{-}O_2S\text{-}A\text{-}NCO \qquad (1)$$

in welcher A einen Arylenrest, beispielsweise einen Phenylenrest, der Substituenten aus der Reihe Alkyl($C_1$-$C_6$), Alkoxy($C_1$-$C_6$), Chlor, Brom und Nitro enthalten kann, oder einen Naphthylenrest bedeutet, indem man auf 1 Mol eines β-Hydroxyäthylsulfonyl-arylamins der allgemeinen Formel (2)

$$HO\text{-}H_2C\text{-}H_2C\text{-}O_2S\text{-}A\text{-}NH_2 \qquad (2)$$

in welcher A die vorstehend genannte Bedeutung hat, in Gegenwart eines Katalysators und gegebenenfalls in einem inerten Lösungsmittel die mindestens stöchiometrische Menge, das heißt, mindestens 2 Mol Thionylchlorid bei Temperaturen von etwa 60 bis etwa 140°C, vorzugsweise von etwa 100 bis etwa 130°C, einwirken läßt und anschließend das entstandene Zwischenprodukt mit der mindestens äquimolaren Menge Phosgen bei Temperaturen von etwa 20 bis etwa 160°C, vorzugsweise etwa 80 bis etwa 130°C, umsetzt.

Als Amine der genannten allgemeinen Formel (2) seien beispielsweise genannt
1-Amino-4-(β-hydroxyäthylsulfonyl)-benzol,
1-Amino-3-(β-hydroxyäthylsulfonyl)-benzol,
1-Amino-2-methoxy-5-(β-hydroxyäthylsulfonyl)-benzol,
1-Amino-2-methoxy-4-(β-hydroxyäthylsulfonyl)-benzol,
1-Amino-4-methoxy-5-(β-hydroxyäthylsulfonyl)-benzol,
1-Amino-2-methyl-5-(β-hydroxyäthylsulfonyl)-benzol,
1-Amino-2-methyl-4-(β-hydroxyäthylsulfonyl)-benzol,
1-Amino-4-methyl-5-(β-hydroxyäthylsulfonyl)-benzol,
1-Amino-2-methoxy-5-methyl-4-(β-hydroxyäthylsulfonyl)-benzol,
1-Amino-2,5-dimethoxy-4-(β-hydroxyäthylsulfonyl)-benzol,
1-Amino-2,4-dimethoxy-5-(β-hydroxyäthylsulfonyl)-benzol,
1-Amino-2-methyl-5-methoxy-4-(β-hydroxyäthylsulfonyl)-benzol,
2-Chlor-1-amino-5-(β-hydroxyäthylsulfonyl)-benzol,
2-Chlor-1-amino-4-(β-hydroxyäthylsulfonyl)-benzol,
2-Brom-1-amino-4-(β-hydroxyäthylsulfonyl)-benzol,
4-Chlor-1-amino-2-methyl-3-(β-hydroxyäthylsulfonyl)-benzol,
2-Chlor-1-amino-5-methyl-4-(β-hydroxyäthylsulfonyl)-benzol,
5-Chlor-1-amino-2-methoxy-4-(β-hydroxyäthylsulfonyl)-benzol,
2-Nitro-1-amino-5-(β-hydroxyäthylsulfonyl)-benzol,
1-Amino-5-(β-hydroxyäthysulfonyl)-naphthalin,
1-Amino-6-(β-hydroxyäthysulfonyl)-naphthalin,
2-Amino -5-(β-hydroxyäthysulfonyl)-naphthalin,
2-Amino-6-(β-hydroxyäthysulfonyl)-naphthalin und
2-Amino-8-(β-hydroxyäthysulfonyl)-naphthalin.

An geeigneten Katalysatoren seien beispielsweise genannt tertiäre Basen, wie beispielsweise Pyridin oder ein Trialkyl($C_1$-$C_6$)-amin, wie beispielsweise Trimethyl- oder Triäthylamin, sowie Verbindungen mit einer N,N-Dialkylcarbonsäureamid-Gruppierung, wie beispielsweise ein N,N-Dialkyl($C_1$-$C_6$)-amid einer aliphatischen Carbonsäure mit 1 bis etwa 20 Kohlenstoffatomen, wie beispielsweise der Ameisen-, Essig-, Propion-, Laurin-, Palmitin- oder Stearinsäure, wie beispielsweise Dimethylformamid oder Diäthylformamid, N,N-Dimethylacetamid oder N,N-Diäthylacetamid, N-Alkyl($C_1$-$C_6$)-pyrollidon, wie beispielsweise N-Methyl-pyrrolidon oder N-Äthyl-pyrrolidon, sowie ein Phosphorsäure-tris-dialkyl($C_1$-$C_6$)amid, wie beispielsweise Phosphorsäure-tris-dimethylamid oder -diäthylamid, oder ein Tetraalkyl($C_1$-$C_6$)-harnstoff, wie beispielsweise Tetramethyl- oder Tetraäthylharnstoff.

Bei der Umsetzung der Aminoverbindungen der genannten allgemeinen Formel (2) mit der zweifach molaren Menge Thionylchlorid ist es vorteilhaft, das Thionylchlorid in einem etwa 20 bis etwa 50 %igen, vorzugsweise einem etwa 20 bis etwa 30 %igen molaren Überschuß einzusetzen. Hierbei ist es zweckmäßig, das Thionylchlorid dem in Lösung oder Suspension in einem inerten organischen Lösungsmittel vorliegenden Amin der genannten Formel (2) bei niedereren Temperaturen, beispielsweise bei etwa 20 bis etwa 60° C, zuzugeben und danach die Temperatur zu steigern. Das ggfs. im Überschuß zugesetzte Thionylchlorid kann vor der Zugabe des Phosgens durch Destillieren mehr oder weniger vollständig entfernt und zurückgewonnen werden.

Bei der Umsetzung des durch Reaktion des Amins der genannten Formel (2) mit dem Thionylchlorid entstandenen Zwischenprodukts, das nicht zwischenisoliert werden muß, mit dem Phosgen ist es zweckmäßig, das Phosgen zunächst bei einer Temperatur zwischen etwa-10° und etwa +60° C, vorzugsweise zwischen etwa

0 und etwa 45° C, einzuleiten und anschließend die Temperatur auf die gewünschte Reaktionstemperatur zu steigern.

Bei dieser Umsetzung gebildetes Thionylchlorid wird zusammen mit überschüssigem Phosgen nach beendeter Reaktion zweckmäßigerweise durch Abdestillieren aus der Isocyanatlösung entfernt und zurückgewonnen.

Die entstandenen Isocyanate der genannten Formel (1), die in hoher Ausbeute anfallen, können durch restloses Abdestillieren des Lösungsmittels in Substanz isoliert werden. Je nach Löslichkeit des jeweiligen Isocyanats kann auch nach Abkühlen der ggfs. auf ein geringeres Volumen eingeengten Reaktionslösung das kristallin ausgefallene Isocyanat abfiltriert werden.

Beim erfindungsgemäßen Verfahren ist es nicht erforderlich, das bei der Einwirkung des Thionylchlorids auf das Amin der genannten Formel (2) entstandene Zwischenprodukt vor dessen Umsetzung mit dem Phosgen zwischenzuisolieren. Die bevorzugte Ausführung erfolgt somit in Form eines Eintopfverfahrens, was einen erheblichen Vorteil darstellt.

Das erfindungsgemäße Verfahren, das zwei Stufen einschließt, wird zweckmäßigerweise bei Normaldruck durchgeführt. Das Verfahren kann jedoch, insbesondere was die zweite Stufe anbelangt, auch bei Überdruck durchgeführt werden.

Geeignete inerte Lösungsmittel sind beispielsweise Chlorbenzol oder o-Dichlorbenzol.

Die Isocyanate der genannten Formel (1) können sowohl in Substanz als auch in Form der anfallenden Reaktionslösung, gegebenenfalls nach Einengen auf ein geringeres Volumen, für Umsetzungen eingesetzt werden.

Die Isocyanate der genannten Formel (1) stellen wertvolle Ausgangsprodukte bei der Farbstoffherstellung dar. Ihre Verwendung für die Herstellung von Reaktivfarbstoffen ist beispielsweise in DE-AS 1 289 930 beschrieben.

Die nachfolgenden Beispiele dienen zur Erläuterung der Erfindung, ohne sie darauf zu beschränken. Die darin genannten Teile sind Gewichtsteile und die Prozentangaben stellen Gewichtsprozente dar, sofern nichts anderes vermerkt ist. Gewichtsteile verhalten sich zu Volumenteilen wie das Kilogramm zum Liter.

**Beispiel 1**

In eine Suspension aus 1 000 Volumenteilen Chlorbenzol, 201 Teilen 1-Amino-3-(β-hydroxyäthylsulfonyl)-benzol und 5 Teilen Dimethylformamid läßt man bei 20 bis 30°C unter Rühren 298 Teile Thionylchlorid einlaufen. Dann wird innerhalb von 3 Stunden auf 125 bis 135°C erhitzt. Bei Normaldruck werden danach etwa 250 Volumenteile abdestilliert und durch 250 Volumenteile frisches Chlorbenzol ersetzt. Nach Abkühlen werden bei 0 bis 10°C 150 Teile Phosgen eingeleitet. Man läßt 8 Stunden bei 0 bis 20°C rühren und steigert dann die Temperatur innerhalb von 4 Stunden bis zur Siedegrenze. Anschließend werden bei Normaldruck etwa 450 Volumenteile abdestilliert. Die verbliebene Lösung, die frei von Phosgen ist, enthält 230 Teile des Isocyanats der Formel

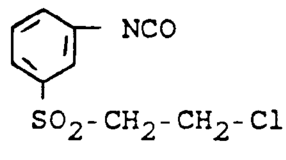

das in reinem Zustand einen Schmelzpunkt von 83°C aufweist.

Man kann die erhaltene Isocyanatlösung (bei 60°C liegt Lösung vor, bei 20°C ist das Arylisocyanat teilweise auskristallisiert) direkt für chemische Umsetzungen verwenden oder das entstandene Isocyanat durch restloses Abdestillieren des Lösungsmittels unter Vakuum als Rückstand in Substanz erhalten.

**Beispiel 2**

Leitet man bei der in Beipsiel 1 beschriebenen Verfahrensweise die angegebene Phosgenmenge nicht bei 0 bis 10°C, sondern bei 45 bis 50°C ein und verfährt im übrigen wie unter Beispiel 1 beschrieben, so erhält man eine Lösung, welche 221 Teile des Isocyanats der Formel

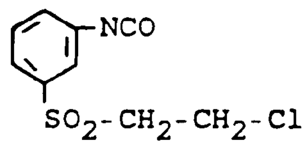

enthält.

**Beispiel 3**

In eine Suspension aus 1 000 Volumenteilen o-Dichlorbenzol, 201 Teilen 1-Amino-3-(β-hydroxyäthylsulfonyl)-benzol und 5 Teilen Dimethylformamid läßt man bei 20 bis 30°C 280 Teile Thionylchlorid einlaufen. Dann wird innerhalb von 4 Stunden auf 140°C hochgeheizt. Anschließend läßt man auf 20°C abkühlen, leitet bei 0 bis 20°C 150 Teile Phosgen ein und läßt 10 Stunden bei 10 bis 30°C Rühren. Darauf wird innerhalb von 4 Stunden auf 140°C hochgeheizt. Man erhält eine Lösung, welche 220 Teile des Isocyanats der Formel

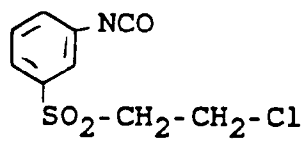

enthält.

Ersetzt man in den Beispielen 1 bis 3 das 1-Amino-3-(β-hydroxyäthylsulfonyl)-benzol durch die äquimolare Menge eines in Spalte 2 der nachstehenden Tabelle aufgeführten Amins der Formel (2) und verfährt im übrigen sinngemäß wie in den Beispielen 1 bis 3 und 18 bis 23 beschrieben, so erhält man die in Spalte 3 angegebenen Isocyanate der Formel (1) mit den in Spalte 4 aufgeführten Schmelzpunkten der reinen Verbindungen in ähnlichen Ausbeuten, wie in den Beispielen 1 bis 3 angegeben.

| Beispiel | Amin der Formel (2) $R_1$= $-SO_2-CH_2-CH_2-OH$ | Isocyanat der Formel (1) $R_2$= $-SO_2-CH_2-CH_2-Cl$ | Schmelzpunkt |
|---|---|---|---|
| 4 | 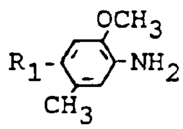 | 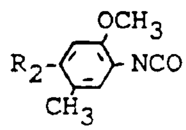 | 130°C |
| 5 | 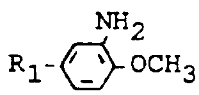 | 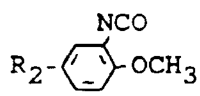 | 122°C |
| 6 | 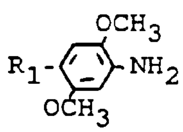 | 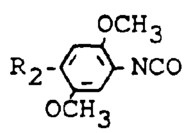 | 104°C |
| 7 | 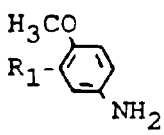 | 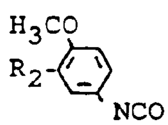 | 76°C |
| 8 | 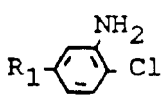 | 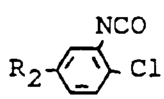 | 103°C |
| 9 | 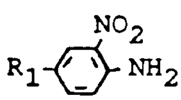 | 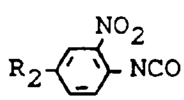 | 101°C |
| 10 | 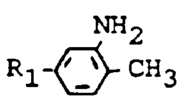 | 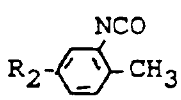 | 89°C |
| 11 | 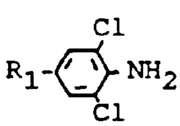 | 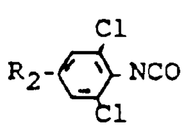 | 97°C |
| 12 | 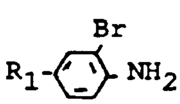 | 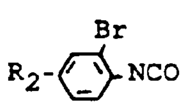 | 119°C |
| 13 | 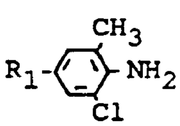 | 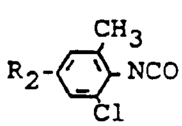 | 120°C |

| Beispiel | Amin der Formel (2) $R_1 = -SO_2-CH_2-CH_2-OH$ | Isocyanat der Formel (1) $R_2 = -SO_2-CH_2-CH_2-Cl$ | Schmelzpunkt |
|---|---|---|---|
| 14 | 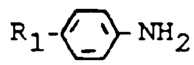 | 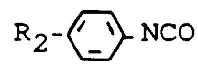 | 100°C |
| 15 | 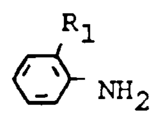 | 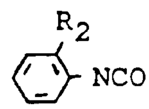 | 193°C |
| 16 | 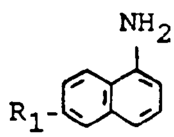 | 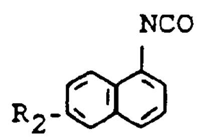 | 130°C |

**Beispiel 17**

In einem mit KPG-Rührer, Thermometer, Destillieraufsatz und beheizbarem Tropftrichter versehenen Kolben wird eine Mischung aus 1 200 Volumenteilen Chlorbenzol, 5 Teilen Dimethylformamid und 300 Teilen Thionylchlorid auf 80°C erwärmt. Dann läßt man innerhalb einer Stunde eine 80 bis 90°C warme Schmelze von 190 Teilen 1-Amino-3-(β-hydroxyäthylsulfonyl)-benzol unter Rühren in die 80 bis 85°C heiße Lösung eintropfen. Man läßt noch 1 Stunde bei 80 bis 85°C rühren und erhöht dann die Temperatur innerhalb von 2 Stunden bis zur Siedegrenze. Danach werden unter Normaldruck 320 Volumenteile in eine Vorlage destilliert.

Man läßt auf 30°C abkühlen und leitet bei 30 bis 50°C innerhalb einer Stunde 200 Teile Phosgen ein. Man läßt noch 2 Stunden bei 30 bis 50°C rühren und steigert dann die Temperatur innerhalb von 4 Stunden bis zur Siedegrenze und entfernt überschüssiges Phosgen und Thionylchlorid durch Abdestillieren. Die verbliebene Lösung enthält 210 Teile des Isocyanats der Formel

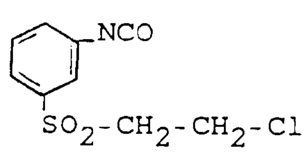

**Beispiel 18**

Ersetzt man in den Beispielen 1 bis 3 das Dimethylformamid durch die äquimolare Menge Pyridin und verfährt im übrigen gemäß den in den Beispielen 1 bis 3 beschriebenen Arbeitsweisen, so erhält man in ähnlichen Ausbeuten wie dort ebenfalls das Isocyanat der Formel

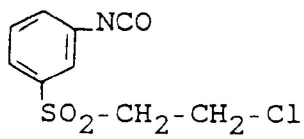

**Beispiel 19**

Ersetzt man in den Beispielen 1 bis 3 das Dimethylformamid durch die äquimolare Menge Triäthylamin und verfährt im übrigen gemäß den in den Beispielen 1 bis 3 beschriebenen Arbeitsweisen, so erhält man in ähnlichen Ausbeuten wie dort ebenfalls das Isocyanat der Formel

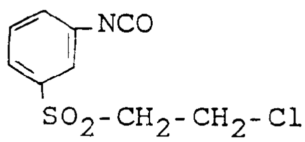

**Beispiel 20**

Ersetzt man in den Beispielen 1 bis 3 das Dimethylformamid durch die äquimolare Menge N.N-Dimethylacetamid und verfährt im übrigen gemäß den in den Beispielen 1 bis 3 beschriebenen Arbeitsweisen, so erhält man in ähnlichen Ausbeuten wie dort ebenfalls das Isocyanat der Formel

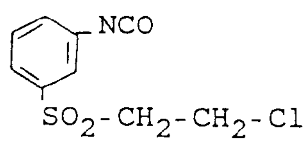

**Beispiel 21**

Ersetzt man in den Beispielen 1 bis 3 das Dimethylformamid durch die äquimolare Menge N.N-Diisobutylacetamid und verfährt im übrigen gemäß den in den Beispielen 1 bis 3 beschriebenen Arbeitsweisen, so erhält man in ähnlichen Ausbeuten wie dort ebenfalls das Isocyanat der Formel

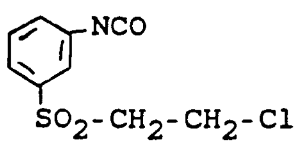

**Beispiel 22**

Ersetzt man in den Beispielen 1 bis 3 das Dimethylformamid durch die äquimolare Menge Phosphorsäure-tris-dimethylamid und verfährt im übrigen gemäß den in den Beispielen 1 bis 3 beschriebenen Arbeitsweisen, so erhält man in ähnlichen Ausbeuten wie dort ebenfalls das Isocyanat der Formel

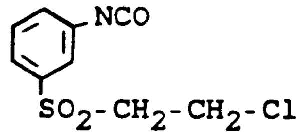

**Beispiel 23**

Ersetzt man in den Beispielen 1 bis 3 das Dimethylformamid durch die äquimolare Menge Tetramethylharnstoff und verfährt im übrigen gemäß den in den Beispielen 1 bis 3 beschriebenen Arbeitsweisen, so erhält man in ähnlichen Ausbeuten wie dort ebenfalls das Isocyanat der Formel

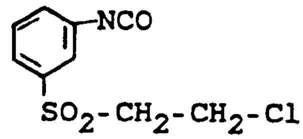

## Patentansprüche

1. Verfahren zur Herstellung von β-Chloräthylsulfonyl-arylisocyanaten der allgemeinen Formel (1)

   $Cl\text{-}H_2C\text{-}H_2C\text{-}O_2S\text{-}A\text{-}NCO$ (1)

   in welcher A einen Arylenrest, der Substituenten aus der Reihe Alkyl($C_1$-$C_6$), Alkoxy($C_1$-$C_6$), Chlor, Brom und Nitro enthalten kann, oder einen Naphthylenrest bedeutet, dadurch gekennzeichnet, daß man auf 1 Mol β-Hydroxyäthylsulfonyl-arylamin der allgemeinen Formel (2)

   $HO\text{-}H_2C\text{-}H_2C\text{-}O_2S\text{-}A\text{-}NH_2$ (2)

   in welcher A die vorstehend genannte Bedeutung hat, in Gegenwart eines Katalysators und gegebenenfalls in einem inerten Lösungsmittel die mindestens stöchiometrische Menge (2 Mol) Thionylchlorid bei Temperaturen von etwa 60 bis etwa 140°C einwirken läßt und anschließend das entstandene Zwischenprodukt mit der mindestens äquimolaren Menge Phosgen bei Temperaturen von etwa 20 bis etwa 160°C umsetzt.

2. Verfahren nach Anspruch 1, dadurch gekennzeichnet, daß man als Katalysator eine tertiäre Base, ein N,N-Dialkyl ($C_1$-$C_6$)-amid einer aliphatischen Carbonsäure mit 1 bis etwa 20 Kohlenstoffatomen, ein Phosphorsäure-tris-dialkyl($C_1$-$C_6$)-amid oder einen Tetraalkyl($C_1$-$C_6$)-harnstoff verwendet.

3. Verfahren nach mindestens einem der Ansprüche 1 und 2, dadurch gekennzeichnet, daß man als Katalysator Pyridin, Trimethylamin, Triäthylamin, Dimethylformamid, Diäthylformamid, N,N-Dimethylacetamid, N,N-Diäthylacetamid, Phosphorsäure-tris-dimethylamid, Phosphorsäure-tris-diäthylamid, Tetramethylharnstoff oder Tetraäthylharnstoff verwendet.

4. Verfahren nach mindestens einem der Ansprüche 1 bis 3, dadurch gekennzeichnet, daß man das Thionylchlorid auf das Amin bei Temperaturen von etwa 100 bis etwa 130°C einwirken läßt.

5. Verfahren nach mindestens einem der Ansprüche 1 bis 4, dadurch gekennzeichnet, daß man die Umsetzung des entstandenen Zwischenprodukts mit dem Phosgen bei Temperaturen von etwa 80 bis etwa 130°C vornimmt.

6. Verfahren nach mindestens einem der Ansprüche 1 bis 5, dadurch gekennzeichnet, daß man die Reaktion mit Thionylchlorid und Phosgen in Monochlorbenzol oder o-Dichlorbenzol als inertem organischen Lösungsmittel durchführt.

7. Verfahren nach mindestens einem der Ansprüche 1 bis 6, dadurch gekennzeichnet, daß man das Verfahren als Eintopfverfahren durchführt.

## Claims

1. A process for the preparation of a β-chloroethyl-sulfonyl-aryl isocyanate of the formula (1)

$$Cl\text{-}H_2C\text{-}H_2C\text{-}O_2S\text{-}A\text{-}NCO \qquad (1)$$

in which A denotes an arylene radical, which can contain substituents from the series comprising alkyl($C_1$-$C_6$), alkoxy($C_1$-$C_6$), chlorine, bromine and nitro, or a naphthylene radical, which comprises allowing at least the stoichiometric amount (2 moles) of thionyl chloride to act on 1 mole of a β-hydroxymethylsulfonyl-arylamine of the formula (2)

$$HO\text{-}H_2C\text{-}H_2C\text{-}O_2S\text{-}A\text{-}NH_2 \qquad (2)$$

in which A has the abovementioned meaning, in the presence of a catalyst and if appropriate in an inert solvent at temperatures of about 60 to about 140°C, and then reacting the resulting intermediate product with at least the equimolar amount of phosgene at temperatures of about 20 to about 160°C.

2. The process as claimed in claim 1, wherein a tertiary base, an N, N-dialkyl($C_1$-$C_6$)-amide of an aliphatic carboxylic acid having 1 to about 20 carbon atoms, a phosphoric ac id tris -dialkyl ($C_1$-$C_6$)amide or a tetraalkyl-($C_1$-$C_6$) -urea is used as the catalyst.

3. The process as claimed in at least one of claims 1 and 2, wherein pyridine, trimethylamine, triethylamine, dimethylformamide,diethylformamide,N,N-dimethylacetamide, N,N-diethylacetamide, phosphoric acid tris-dimethylamide, phosphoric acid tris-diethylamide, tetramethylurea or tetraethylurea is used as the catalyst.

4. The process as claimed in at least one of claims 1 to 3, wherein the thionyl chloride is allowed to act on the amine at temperatures of about 100 to about 130°C.

5. The process as claimed in at least one of claims 1 to 4, wherein the reaction of the resulting intermediate product with the phosgene is carried out at temperatures of about 80 to about 130°C.

6. The process as claimed in at least one of claims 1 to 5, wherein the reaction is carried out with thionyl chloride and phosgene in monochlorobenzene or o-dichlorobenzene as the inert organic solvent.

7. The process as claimed in at least one of claims 1 to 6, wherein the process is carried out as a one-pot process.

## Revendications

1. Procédé de préparation d'isocyanates de β-chloréthylsulfonyl-aryles de formule générale 1 ci-dessous :

$$Cl\text{-}H_2C\text{-}H_2C\text{-}O_2S\text{-}A\text{-}NCO \qquad (1)$$

(dans laquelle A désigne un radical arylène pouvant avoir des substituants de la série d'alkyles et alcoxy en $C_1$-$C_6$ chacun, du chlore, du brome et du groupe nitro, ou un radical naphtylène), procédé caractérisé en ce que l'on fait réagir avec 1 mol d'une β-hydroxyéthyl-sulfonylarylamine de formule générale 2 :

$$HO\text{-}H_2C\text{-}H_2C\text{-}O_2S\text{-}A\text{-}NH_2 \qquad (2)$$

A ayant la signification ci-dessus, en présence d'un catalyseur et le cas échéant d'un solvant inerte, une proportion au moins stoechiométrique, c'est-à-dire d'au moins 2 mol, de chlorure de thionyle, à des températures d'environ 60 à 140°C, puis on fait réagir le produit intermédiaire ainsi formé avec une proportion au moins équimolaire de phosgène à des températures d'environ 20 à 160°C.

2. Procédé selon la revendication 1, caractérisé en ce que le catalyseur employé est une base tertiaire, un N,N-dialkyl($C_1$-$C_6$)-amide d'un acide carboxylique aliphatique pouvant avoir de 1 à environ 20 atomes de carbone, un tris-dialkyl($C_1$-$C_6$)-amide de l'acide phosphorique ou une tétralkyl($C_1$-$C_6$)-urée.

3. Procédé selon au moins l'une des revendications 1 et 2, caractérisé en ce que le catalyseur employé est de la pyridine, de la triméthylamine, de la triéthylamine, du diméthylformamide, du diéthylformamide, du N,N-diméthylacétamide, du N,N-diéthylacétamide, le tris-diméthylamide de l'acide phosphorique, le tris-diéthylamide de l'acide phosphorique, la tétraméthylurée ou encore la tétraéthylurée.

4. Procédé selon au moins l'une des revendications 1 à 3, caractérisé en ce que l'on fait réagir le chlorure de thionyle avec l'amine à des températures d'environ 100 à 130°C.

5. Procédé selon au moins l'une des revendications 1 à 4, caractérisé en ce que l'on effectue à des températures d'environ 80 à 130°C la réaction du produit intermédiaire formé avec le phosgène.

6. Procédé selon au moins l'une des revendications 1 à 5, caractérisé en ce que l'on effectue les réactions avec le chlorure de thionyle et le phosgène dans du monochlorobenzène ou de l'ortho-dichlorobenzène comme solvant organique inerte.

7. Procédé selon au moins l'une des revendications 1 à 6, caractérisé en ce que l'on effectue les deux réactions dans le même récipient, sans isoler le produit intermédiaire formé par la première réaction.